(19)

**Europäisches Patentamt**
**European Patent Office**
**Office européen des brevets**

(11) **EP 4 242 662 A1**

(12) **EUROPEAN PATENT APPLICATION**

(43) Date of publication:
**13.09.2023 Bulletin 2023/37**

(21) Application number: **22161689.9**

(22) Date of filing: **11.03.2022**

(51) International Patent Classification (IPC):
**G01N 33/68** (2006.01)

(52) Cooperative Patent Classification (CPC):
**G01N 33/6896;** G01N 2800/2835; G01N 2800/52;
G01N 2800/60

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **National University of Ireland Galway Galway (IE)**

(72) Inventor: **The designation of the inventor has not yet been filed**

(74) Representative: **Barker Brettell LLP**
**100 Hagley Road**
**Edgbaston**
**Birmingham B16 8QQ (GB)**

(54) **DIAGNOSTIC MARKERS OF PARKINSON'S DISEASE**

(57)  The invention relates to a method of determining whether an individual has Parkinson's disease, the method comprising: determining the level of markers in a bodily fluid sample from the individual, wherein the markers comprise Protein disulphide isomerase A1 (PDIA1), Protein disulphide isomerase A3 (PDIA3), Mesencephalic astrocyte-derived neurotrophic factor (MANF) and clusterin; wherein an increase in the level of PDIA1, MANF, and clusterin, and decrease in the level of PDIA3 relative to a reference level in combination with the individual's age and gender, is indicative of the individual having Parkinson's disease; and associated compositions, uses and methods.

**Figure 1**

EP 4 242 662 A1

**Description**

The present invention relates to methods of determining whether an individual has Parkinson's disease; and related treatments, compositions and kits.

Parkinson's disease (PD) is the second most common neurodegenerative disorder of the central nervous system. It is characterized by a progressive loss of dopaminergic neurons in substantia nigra pars compacta and the accumulation of misfolded α-synuclein fibrils in glial and neuronal inclusions, called Lewy bodies [1]. Making an accurate diagnosis of PD is difficult, time-consuming and is based on clinical assessment of symptoms, medical history, physical and neurological examination, and response to the drug Levodopa. Imaging methods such as magnetic resonance imaging (MRI), photon emission tomography (PET) and single-photon emission computed tomography (SPECT) are used to exclude conditions not associated with dopamine deficiency [1]. Unfortunately, they are costly and some require exposure of patients to radiation. Furthermore, the frequency of misdiagnosis of PD is high due to overlap of symptoms with other disorders, such as multiple system atrophy, progressive supranuclear palsy, corticobasal syndrome or dementia with Lewy bodies [43]. Therefore, reliable diagnostic tests monitoring pathological processes associated with PD are urgently needed.

An aim of the present invention is to provide a fast, convenient test supporting diagnosis of Parkinson's disease based on clinical symptoms.

According to a first aspect of the present invention, there is provided a method of determining whether an individual has Parkinson's disease, the method comprising:

determining the level of markers in a bodily fluid sample from the individual, wherein the markers comprise or consist of Protein disulphide isomerase A1 (PDIA1), Protein disulphide isomerase A3 (PDIA3), Mesencephalic astrocyte-derived neurotrophic factor (MANF) and clusterin;
wherein an increase in the level of PDIA1, MANF, and clusterin, and decrease in the level of PDIA3 relative to a reference level in combination with the individual's age and gender, is indicative of the individual having Parkinson's disease.

The probability that an individual has Parkinson's disease may be determined by the calculation:

$$P(PD)$$
$$= \frac{\exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}{1 + \exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}$$

wherein Protein A is PDIA1, Protein C is clusterin, Protein E is MANF and Protein F is PDIA3 and these proteins are the measured biomarker levels, age is the individual's age and gender is assigned 1 for female or 0 for male.

Advantageously, a panel of biomarkers is provided which is an economic, relatively non-invasive, and rapid method that is readily used by clinicians for the diagnosis of various other disorders. The panel of biomarkers can aid in the identification of Parkinson's disease in patients with mild to moderate symptoms according to stages 2-3 of the Hoehn and Yahr scale.

The Hoehn and Yahr scale is a widely used scale to categorise the severity of Parkinson's disease, which the skilled person would be familiar [44].

**The individual**

In an embodiment according to the first aspect, the individual may be at Hoehn and Yahr stage 2 or 3 of Parkinson's disease. The individual may be exhibiting clinical features of Parkinson's disease.

An individual with Parkinson's disease at Hoehn and Yahr stage 2 is characterised by symptoms on both sides of the body or at the midline without impairment to balance. An individual with Parkinson's disease at Hoehn and Yahr stage 3 is characterised by mild to moderate bilateral disease with some postural instability and slowness of movement. However, an individual at Hoehn and Yahr stage 3 is still fully independent in their daily living activities.

In one embodiment, the method may further comprise the assessment of clinical features of Parkinson's disease. The clinical features of Parkinson's disease may comprise bradykinesia in combination with one or more, or all, of the clinical features selected from the group comprising: rest tremor, rigidity, postural instability, hypomimia, dysarthria, dysphagia, sialorrhoea, decreased arm swing, shuffling gait, festination, difficulty arising from chair, difficulty turning in bed, micrographia, difficulty cutting food, reduced hygiene, slow activities of daily living, glabellar reflex, blepharospasm, dystonia, striatal deformity, scoliosis, camptocormia, cognitive impairment, bradyphrenia, tip-of-the-tongue phenomenon, depression, apathy, anhedonia, fatigue, other behavioural and psychiatric problems, sensory symptoms (such as anosmia, ageusia, pain (shoulder, back) or paresthesias), dysautonomia (such as orthostatic hypotension, constipation, urinary and sexual dysfunction, abnormal sweating, seborrhoea), weight loss, sleep disorders (such as REM behaviour disorder, vivid dreams, daytime drowsiness, sleep fragmentation, restless legs syndrome), and/or response to high-dose levodopa. The skilled person, such as a clinician/medical professional, will be familiar with the number and combination of clinical features that may be used to assess the presence and/or progression of Parkinson's disease.

Advantageously, the combination of traditional clinical examination and the method of the invention reduces misdiagnosis of Parkinson's disease compared to clinical examination alone.

In one embodiment, the individual is a human. In another embodiment, the individual is an adult human, such as a human over the age of 30. In another embodiment, the individual is a human over the age of 35. In another embodiment, the individual is a human over the age of 36. In another embodiment, the individual is a human over the age of 37. In another embodiment, the individual is a human over the age of 38. In another embodiment, the individual is a human over the age of 39. In another embodiment, the individual is a human over the age of 40. In another embodiment, the individual is a human between the ages of 30 and 100. In another embodiment, the individual is a human between the ages of 30 and 90. In another embodiment, the individual is a human between the ages of 35 and 100. In another embodiment, the individual is a human between the ages of 35 and 90. In another embodiment, the individual is a human between the ages of 40 and 100. In another embodiment, the individual is a human between the ages of 40 and 90. In a preferred embodiment, the individual is a human between the ages of 39 and 88.

## The level of marker increase/decrease

The increase or decrease in the level of the marker may be relative to an average level of the marker in a general population that are not, or not known to be, afflicted with Parkinson's disease. The increase or decrease may be a statistically significant increase or decrease.

References herein to an increase or decrease in the level of a given marker is intended to refer to an increase or decrease in determined marker level relative to the mean value of a non-Parkinson's group (i.e. the mean value of a normal population that does not have Parkinson's disease).

## Determining whether the subject has Parkinson's disease

The probability that an individual has Parkinson's disease may be determined by the calculation:

$$P(PD)$$
$$= \frac{\exp(-5.9197 + 1.2402\,Protein\,A + 0.01192\,Protein\,C + 4.2073\,Protein\,E - 4.2548\,Protein\,F + 0.0264\,Age - 1.5851\,Gender)}{1 + \exp(-5.9197 + 1.2402\,Protein\,A + 0.01192\,Protein\,C + 4.2073\,Protein\,E - 4.2548\,Protein\,F + 0.0264\,Age - 1.5851\,Gender)}$$

wherein Protein A (PDIA1), Protein C (clusterin), Protein E (MANF) and Protein F (PDIA3) are the measured biomarker levels, age is the individual's age (years) and gender is assigned 1 for female or 0 for male.

The odds of PD for a female is 80% lower than a male individual, and for every one unit (ng/ml) increase in protein E (MANF), the odds of Parkinson's disease will increase by 6617%. In addition, for every one unit (ng/ml) increase in protein A PDIA1, the odds of Parkinson's disease will increase by 245%. For every one unit (μg/ml) increase in protein C (clusterin), the odds of Parkinson's disease will increase by 1%. For every one unit (ng/ml) increase in protein F (PDIA3), the odds of Parkinson's disease will decrease by 98%. For every one year increase in Age, the odds of Parkinson's disease will increase by 2%.

## Interventions/Actions

The methods of the invention may be used, for example, for any one or more of the following: to diagnose an individual with Parkinson's disease; to advise on the prognosis of an individual with suspected Parkinson's disease; to advise on treatment options for an individual with Parkinson's disease, for example to treat current symptoms or to slow disease progression. The presence, or level, of the markers may be used to stratify patients. This stratification may be used to decide the appropriate treatment.

Information regarding the Parkinson's disease status of an individual may be relayed to a third party, such as a doctor, other medical professional, pharmacist, or other interested party. This information may be relayed digitally, for example via email, SMS or other digital means.

The method may further comprise selecting the individual for therapeutic intervention and/or follow-up check if the individual is diagnosed with Parkinson's disease. In another embodiment, the method may further comprise administering a therapeutic or preventative medication to the individual, if the individual is diagnosed with Parkinson's disease. Additionally or alternatively, the method may further comprise providing supportive therapies including one or more of physiotherapy, occupational therapy, speech and language therapy and diet advice, if the individual is diagnosed with Parkinson's disease. Additionally or alternatively, the method may further comprise referring the individual for, or conducting, surgery arranged to alleviate some of the symptoms of Parkinson's disease (such as deep brain stimulation), if the individual is diagnosed with Parkinson's disease.

**Medication**

The therapeutic or preventative medication may comprise dopamine receptor activators, dopamine precursors, inhibitors of dopamine degradation, indirect dopamine receptor activators. The therapeutic or preventative medication may comprise levodopa, carbidopa-levodopa, duodopa, dopamine agonists (such as Pramipexole (Mirapex), Ropinirole (Requip) and Rotigotine (Neupro). Apomorphine (Apokyn)), monoamine oxidase-B inhibitors (such as selegiline (Zelapar), rasagiline (Azilect) and safinamide (Xadago)), catechol-O-methyltransferase inhibitors (such as Entacapone (Comtan), opicapone (Ongentys) and Tolcapone (Tasmar)), anticholinergics (such as benztropine (Cogentin) or trihexyphenidyl), Amantadine, or antagonists of adenosine A2A receptors (such as Istradefylline); or combinations thereof.

The skilled person will recognise that the medication can be administered, or arranged to be administered, at an appropriate dose via the appropriate administration route. In one embodiment, the medication may comprise or consist of a therapeutically or prophylactically effective amount of the medication.

**Marker level determination**

In one embodiment, the presence, absence, or level of a marker may be determined by any suitable assay. The skilled person will recognise there are a number of methods and technologies available to determine the presence and/or level of a marker, such as a protein marker.

Determining the level of a marker may comprise quantifying the presence of the marker in the sample. The level of marker in the sample may be compared relative to a control sample, or a predetermined standard level.

Determining the level of a marker may comprise binding a marker with one or more probes. The method may further comprise detecting the act of binding of the probe(s) to the marker or detecting the level of bound probe-marker complexes.

In one embodiment, determining the level of a marker may comprise conducting an enzyme-linked immunosorbent assay (ELISA). The ELISA may comprise a competitive immunoassay, sandwich immunoassay or antibody capture. In particular, an ELISA may be used to determine the level of one or more markers in the sample. The ELISA may comprise a multiplexed ELISA. The multiplexed ELISA may comprise planar antibody arrays, Biochip Array Technology (BAT) multiplexed assay, membrane antibody arrays, or qualitative glass slide-based antibody arrays. The ELISA may be suspension-based, such as bead-based multiplex flow cytometry assay. The ELISA may be direct or indirect.

In another embodiment, the level of marker may be determined by aptamer-based ELASA (enzyme-linked apta-sorbent assay).

In another embodiment, the level of marker may be determined by western blot.

In another embodiment, the level of a marker may be determined by detecting the marker directly, for example by mass-spectrometry. The mass-spectrometry may comprise liquid chromatography mass-spectrometry. The mass-spectrometry may comprise matrix assisted laser desorption ionization-time of flight mass-spectrometry (MALDI-TOF). The mass-spectrometry may comprise two-dimensional gel electrophoresis mass-spectrometry. The mass-spectrometry may comprise selective reaction monitoring mass-spectrometry. The mass-spectrometry may comprise tandem mass-spectrometry.

In another embodiment, the level of a marker may be determined by a Proximity Extension Assay (PEA) (e.g. by Olink Proteomics™). For example, PEA may comprise the provision of a matched pair of proximity probes, for example comprising a pair of antibodies, or fragments thereof, arranged to bind the targeted marker, and both linked to respective oligonucleotides arranged to hybridise to each other. As a result of the proximity probes binding to the same targeted marker, the probes can come in close proximity and hybridize to each other. The addition of a DNA polymerase can lead to an extension of the hybridizing oligo, bound to one of the probes, creating a DNA amplicon that can subsequently be detected and quantified, for example by quantitative real-time PCR.

**Probes**

The probe (according to any aspect of the invention) may be a binding agent that is capable of specific/selective binding to a protein marker of interest. The binding agent may comprise or consist of a polypeptide and/or nucleic acid, such as DNA. The probe may comprise or consist of an antibody, an antibody variant or memetic, or a binding-fragment thereof. In another embodiment, the probe may comprise or consist of an aptamer. In one embodiment, the probe for a given marker is a polyclonal or monoclonal antibody, or fragment thereof. The antibody, or fragment thereof, may be of any mammalian species, such as human, simian, porcine, camelid or rabbit.

In one embodiment, the probes are immobilised on a substrate. One or more, or all of the probes may be anchored to a surface, such as the surface of a solid substrate. The solid substrate may be a plate, such as a microwell plate. In another embodiment, the solid substrate may be a particle, such as a nano- or micro-particle. In one embodiment the solid substrate is a bead.

In one embodiment, the probe may comprise a tag identification and/or capture. The tag may comprise a fluorescent molecule, or an enzyme. In another embodiment, the probe may be radiolabelled.

Where ELISA, or similar assay, is used, the probe may be a primary antibody for binding to the target, and a secondary tagged-antibody probe may be provided for binding to the primary antibody or the marker for detection.

**The sample**

In one embodiment the sample may be an extracellular fluid sample. The sample may be a blood sample. The sample may be a serum or a plasma sample. The sample may be a cerebrospinal fluid sample. The sample may be a saliva sample. The sample may be a mucus sample. The sample may be a urine sample.

The sample may be taken/obtained from the individual in the method of the invention. Alternatively, the sample may be provided (previously obtained, for example by a third party). The sample may be fresh, such as less than 1 day from withdrawal. Alternatively, the sample may be a stored sample, for example that has been frozen or refrigerated.

Some or all of the steps of the method(s) of the invention may be carried out *in vitro.*

**Other aspects**

According to another aspect of the present invention, there is provided a composition comprising a plurality of probes capable of binding to protein markers in a bodily fluid sample, wherein the protein markers comprise PDIA1, PDIA3, MANF, and clusterin.

The composition(s) according to the invention may comprise a probe species for each marker to be bound (i.e. each protein may have its own corresponding probe arranged to bind thereto). The plurality of probes may be different to each other (i.e. may be structurally different and bind different proteins/markers or different non-competing epitopes on the same protein/marker).

The probes may be provided as a panel of probes anchored to a surface.

The probes may be capable of selectively binding to two, three, or more of the markers.

According to another aspect of the present invention, there is provided a method of detecting the level of proteins comprising PDIA1, PDIA3, MANF and clusterin, in a bodily fluid sample from an individual; and optionally the bodily fluid sample may be of an individual with suspected Parkinson's disease.

In one embodiment according to any aspect of the invention, all of the listed proteins are detected, or attempted to be detected. In one embodiment according to any of the aspects of the invention, the level of all of the listed proteins is determined.

According to another aspect of the present invention, there is provided a method for treating an individual with Parkinson's disease, the method comprising the steps of:

determining whether the individual has Parkinson's disease by:
obtaining or having obtained a sample from the individual; and performing or having performed the method according to the invention herein to determine if the individual has Parkinson's disease; and
if the individual is found to have Parkinson's disease, then administering medication as described herein to the individual and/or surgically treating the individual.

According to another aspect of the present invention, there is provided a method for treating an individual diagnosed with Parkinson's disease, the method comprising the steps of:

receiving results of a test performed according to the method of the invention herein to determine if the individual has Parkinson's disease; and
if the individual is found to have Parkinson's disease, then administering medication as described herein to the individual and/or surgically treating the individual.

According to another aspect of the present invention, there is provided a method for treating an individual to slow the onset or progression of Parkinson's disease, the method comprising the steps of:

determining whether the individual has Parkinson's disease by:

obtaining or having obtained a sample from the individual; and performing or having performed the method according to the invention herein to determine if the individual has Parkinson's disease; and

if the individual is determined to have Parkinson's disease, then administering medication as described herein to the individual and/or surgically treating the individual.

Surgically treating the individual may comprise the implantation of a deep brain stimulation implant.

According to another aspect of the present invention, there is provided the use of PDIA1, PDIA3, MANF and clusterin, as markers in a bodily fluid sample from an individual for diagnosis of Parkinson's disease.

The use may comprise determining the levels of the proteins in a bodily fluid sample from the individual. The individual may be an individual suspected to have Parkinson's disease. The use of the biomarkers may be in combination with, or by reference to, the determination of the individual's age and gender.

The use may comprise determining the probability that an individual has Parkinson's disease, which may be determined by the calculation:

$$P(PD)$$
$$= \frac{\exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}{1 + \exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}$$

wherein Protein A (PDIA1), Protein C (clusterin), Protein E (MANF) and Protein F (PDIA3) are the measured biomarker levels, age is the individuals age and Gender is assigned 1 for female or 0 for male.

## Definitions

The stages of Parkinson's disease may be classified by the Hoehn and Yahr scale as follows, for which the skilled person will be familiar [44]:

Stage 0 - No signs of disease
Stage 1 - Symptoms on one side only (unilateral)
Stage 1.5 - Symptoms unilateral and also involving the neck and spine
Stage 2 - Symptoms on both sides but no impairment of balance
Stage 2.5 - Mild symptoms on both sides, with recovery when the 'pull' test is given (the doctor stands behind the person and asks them to maintain their balance when pulled backwards)
Stage 3 - Balance impairment, mild to moderate disease, physically independent
Stage 4 - Severe disability, but still able to walk or stand unassisted
Stage 5 - Needing a wheelchair or bedridden unless assisted.

The term "preventative medication" refers to medication which has been demonstrated to slow disease progression.

A "prophylactically effective amount" interchangeable with 'therapeutically effective amount', or 'effective amount', or 'therapeutically effective', as used herein, refers to that amount which provides a therapeutic or preventative effect for a given condition and administration regimen. This is a predetermined quantity of active material calculated to produce a desired therapeutic effect in association with the required additive and diluent, i.e. a carrier or administration vehicle. Further, it is intended to mean an amount sufficient to reduce and most preferably prevent, a clinically significant deficit in the activity, function and response of the individual. Alternatively, a therapeutically effective amount is sufficient to cause an improvement in a clinically significant condition in an individual. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical worker based on patient/ individual characteristics, such as age, weight, sex, condition, complications, other diseases, etc., as is well known in the art.

The term "characteristic motor symptoms" refers to the classical clinical symptoms used to diagnose Parkinson's disease which include one or more of tremor, bradykinesia, rigidity, impaired posture and balance, loss of automatic movements, speech changes and writing changes.

By "antibody" we include substantially intact antibody molecules, as well as chimeric antibodies, human antibodies, humanised antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments, antibody mimetics, and derivatives of the same. In particular, the term "antibody" as used herein refers to immunoglobulin molecules and immunologically active portions of immunoglobulin molecules, i.e., molecules that contain an antigen binding site that specifically binds an antigen, whether natural or partly or wholly synthetically produced. The term also covers any polypeptide or protein having a binding domain which is, or is homologous to, an antibody binding domain. These can be derived from natural sources, or they may be partly or wholly synthetically produced. Examples of antibodies are the immunoglobulin isotypes (e.g., IgG, IgE, IgM, IgD and IgA) and their isotypic subclasses; fragments which comprise an antigen binding domain such as Fab, scFv, Fv, dAb, Fd; and diabodies. Antibodies may be polyclonal or monoclonal. A monoclonal antibody may be referred to as a "mAb".

It has been shown that fragments of a whole antibody can perform the function of binding antigens. Examples of binding fragments of the invention are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment which consists of a VH domain; (v) isolated CDR regions; (vi) F(ab')2 fragments, a bivalent fragment comprising two linked Fab fragments; (vii) single chain Fv molecules (scFv), wherein a VH domain and a VL domain are linked by a peptide linker which allows the two domains to associate to form an antigen binding site; (viii) bispecific single chain Fv dimers and; (ix) "diabodies", multivalent or multispecific fragments constructed by gene fusion.

The markers listed herein may include variants of the markers, for example variants having natural mutations/polymorphisms in a population. It is understood that reference to protein or nucleic acid "variants", is understood to mean a protein or nucleic acid sequence that has at least 70%, 80%, 90%, 95%, 98%, 99%, 99.9% identity with the sequence of the fore mentioned protein or nucleic acid. The percentage identity may be calculated under standard NCBI blast p/n alignment parameters. "Variants" may also include truncations of a protein or nucleic acid sequence. Variants may include biomarker listed herein comprising the same sequence, but comprising or consisting of 1, 2, 3, 4, 5, 6, 7, 8, 9, or 10, or more modifications, such as substitutions, deletions, additions of nucleotides, bases or amino acids. Variants may also comprise redundant/degenerate codon variations. Variants may also include proteins with post-translational modifications, including but not limited to phosphorylation, ubiquitination, glycosylation. The skilled person will recognise that a protein marker could be linear (inactive, denatured), misfolded or native (folded properly). Therefore, variants in the folding status of the marker may be encompassed herein.

The markers herein may be identified as follows:

Clusterin : UniProtKB - P10909;
Mesencephalic astrocyte-derived neurotrophic factor (MANF): UniProtKB - P55145;
Protein disulphide isomerase A1 (PDIA1) : UniProtKB - P07237; and
Protein disulphide isomerase A3 (PDIA3) : UniProtKB - P30101.

The skilled person will understand that optional features of one embodiment or aspect of the invention may be applicable, where appropriate, to other embodiments or aspects of the invention.

Embodiments of the invention will now be described in more detail, by way of example only, with reference to the accompanying drawings.

**Figure 1:** Serum concentrations of a) PDIA1, b) PDIA3, c) MANF, d) GRP78, e) calreticulin and f) clusterin plotted as individual values in indicated diagnostic groups.

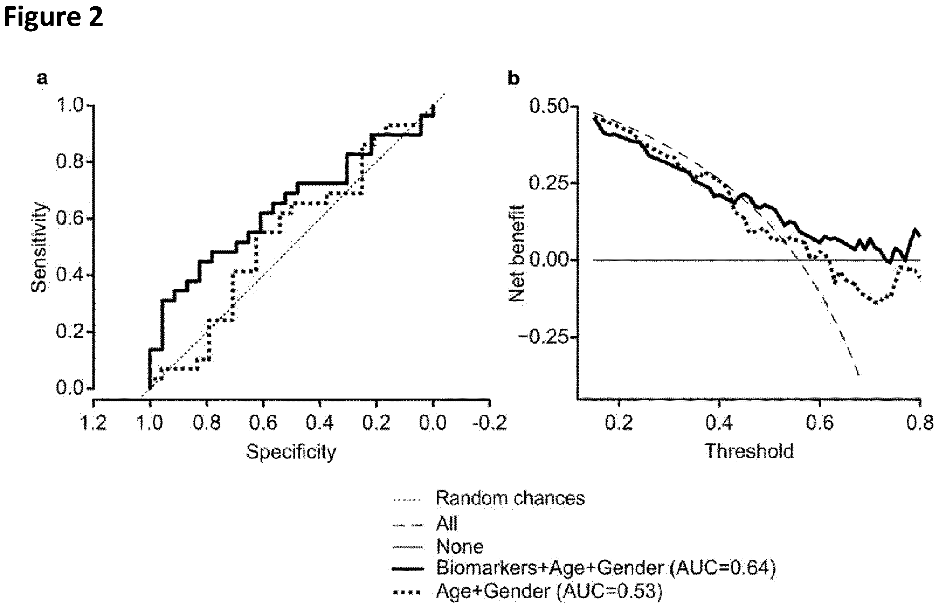

**Figure 2:** a) Receiver Operating Characteristic (ROC) curve and b) decision curve analysis of PD versus non-PD.

**Figure 3:** The effect of $\alpha$-synuclein and pathological $\alpha$-synuclein addition into biomarker panel on a,c,e) Receiver Operating Characteristic (ROC) curve and b,d,f) decision curve analysis of PD versus non-PD.

**Example 1: Endoplasmic reticulum stress-related chaperones as potential serum biomarkers for Parkinson's Disease**

Neuropathological examination of post-mortem brain tissues from PD patients revealed a strong association of PD pathophysiology with stress of endoplasmic reticulum (ER) [2][3][4][5]. Studies using animal and cellular models of PD, including patient-derived induced pluripotent stem cells, indicate that the disruption of ER function is an early event in PD pathogenesis contributing to neurodegeneration. ER is a cellular organelle which plays a central role in controlling intracellular proteostasis and maintains extracellular proteome integrity. Accumulation of misfolded and unfolded proteins drives ER stress and triggers the Unfolded Protein Response (UPR) required to restore proteostasis. If ER stress is too severe the UPR signaling switches to promote neurodegeneration. The UPR leads to upregulation of ER resident chaperones facilitating proper folding and post-translational modifications of transmembrane and secretory proteins. At the same time it reduces release of some prominent extracellular chaperones [10].

An emerging area in the research literature is a systemic communication of stress signals between neurons and distal tissues to maintain nonautonomous control of homeostasis [11][12][13]. One way in which this can be achieved is through the release of cellular stress proteins by neurons which reach the bloodstream and circulate throughout the body [14][15]. This offers the opportunity to develop blood-based biomarkers of neurodegeneration. In this pilot study we assessed serum levels of ER stress-regulated chaperones: PDIA1, PDIA3, GRP78, calreticulin, clusterin and Mesencephalic Astrocyte-Derived Neurotrophic Factor (MANF) in PD patients and non-PD controls. We identified a panel of 4 novel biomarkers which allowed for discrimination between two diagnostic groups.

## Methods:

### Participant recruitment and assessment:

This cross-sectional pilot study was approved by University Hospital Galway Research Ethics committee and written informed consent was obtained from the subjects. Blood samples were collected from 29 idiopathic Parkinson's Disease patients and 24 non-PD controls. Patients diagnosed with Parkinson's Disease by a movement disorder specialist, in accordance with current movement disorders criteria [43], before onset of the study were recruited from a movement disorders specialist clinic in tertiary referral centre. Hoehn & Yahr (H&Y) staging and Mini-Mental State Examination [MMSE] score assessments were completed for enrolled patient on the day of blood collection. Patients were eligible for inclusion if they were diagnosed with idiopathic Parkinson's disease, with no signs of dementia (MMSE score$\geq$24), and moderate disease severity (H&Y stage 2 and 3). Clinical data, such as disease duration and medication, were obtained. Unrelated non-PD subjects or spouses of patients were enrolled from a local community. Volunteers with neurological disorders or a family history of neurodegenerative diseases and on cytotoxic medication were excluded. The demographic and clinical characteristics of the enrolled subjects listed in the table 1 confirm the similar distribution of age and sex between the diagnostic groups.

Table 1 The demographic and clinical characteristics of the enrolled subjects

|  | PD (n=29) | Non-PD (n=24) | P value |
|---|---|---|---|
| Age, mean (range), years | 68.5 (43-83) | 64.3 (39-88) | 0.15 (ns) |
| Gender (F/M) | 13/16 | 15/9 | 0.21 (ns) |
| MMSE score, mean (SD) | 28.73 (1.55) | Nd | nd |
| Y&H stage (number per stage 2/3) | 12/17 | n/a | nd |
| PD duration, mean (SD), months | 124.4 (60.09) | n/a | nd |

### Collection and processing of human serum samples

Venous blood was collected by a trained phlebotomist into BD Vacutainer® serum tubes (Becton, Dickinson, # 367895) containing silica for clot activation. Samples were left to coagulate in a dark at room temperature for 1 h. Tubes were centrifuged at 1,300 x g for 10 min. Serum was collected, aliquoted into polypropylene 0.5 ml tubes and stored at -80 °C within less than 50 minutes from centrifugation. Serum samples were validated for hemolysis.

### Enzyme-linked immunosorbent assay (ELISA)

Protein concentrations in serum were measured by enzyme-linked immunosorbent assays: Protein Disulfide-isomerase A1 (PDIA1) (Abbexa, Cambridge, UK; abx152685), MANF (Abcam, Cambridge, UK; ab215417), PDIA3 (Abbexa; abx252930), GRP78 (Enzo, NY, USA; ADI-900-214), Calreticulin (Abbexa; abx250954), Clusterin (BioVendor, Heidelberg, Germany; RD194034200R), $\alpha$-synuclein (Invitrogen, CA, USA; KHB0061), patho-synuclein (Analytic-Jena, Jena, Germany; BM-847-0104000108) according to the manufacturer's protocols.

### Statistical analysis:

Quantitative variables were compared using unpaired two-tailed t test with Welch's correction. Patient information including serum levels of ER stress-regulated chaperones were used to build a statistical model to predict Parkinson's disease. Biomarker concentrations have been adjusted between plates for those biomarkers that have significant difference between the plate means.

The stepwise variable selection technique [16] was used to identify a combination of biomarkers which could distinguish Parkinson's disease patients from control group. The effects of selected biomarkers were modelled using logistic regression [17]. 10-fold cross-validation [18] was used for Internal validation of the models to confirm that no patient was used to both develop and test the model. 10-fold cross validation involves randomly dividing the data into ten evenly sized subgroups (fold). The data was used from the first nine folds for modelling and applied to the tenth fold as the validation set. The model building, and validation process were repeated ten times with each fold of patients used once as the validation set. Accuracy of the models was determined using the area under the curve (AUC) calculated from the Receiver operator curve [19] by plotting the sensitivity and specificity at each of its risk thresholds. The closer the AUC value to 1, the better the panel of biomarkers can distinguish Parkinson disease patients from control group. Decision-curve analysis [6] was also undertaken to examine the potential net benefit of the application of each model where a higher net benefit value shows improvement in disease detection.

Thirty-one percentage of data for oligomerized synuclein were below the detection limit of the analytical procedure. These data are censored, which are different from missing values as they lie between zero and the detection limit. Cohen's method [20] was used for the imputation of data below the detection limit considering the sample size and the percentage of censoring. The data were imputed using the inverse cumulative normal distribution with the adjusted sample mean and standard deviation using the maximum likelihood estimation.

**Results:**

**Comparison of biomarker candidate protein levels between non-PD and PD groups**

Chaperones PDIA1, PDIA3, MANF, GRP78, calreticulin and clusterin were selected for validation as potential PD biomarkers. All of selected candidates, except clusterin, are ER resident proteins during homeostasis but can be released from cells upon ER stress [21][22][23][24]. Contrary, clusterin is an extracellular chaperone and can be retained inside cells when ER stress is induced [10].

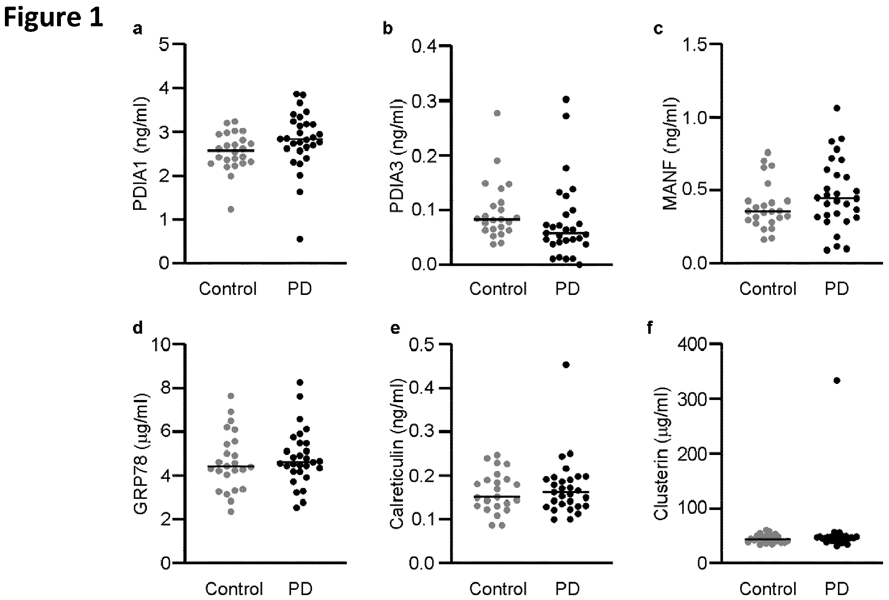

Analysis of protein concentration in serum revealed that the levels of PDIA1 (p=0.096), MANF (p=0.17), and clusterin (p=0.31) were higher in PD patients, PDIA3 (p=0.27) levels were reduced, while GRP78 (p=0.63) and calreticulin (p=0.56) levels were similar in both groups (Table 2, Figure 1).

Table 2:

| | Non-PD (n=24) | PD (n=29) |
|---|---|---|
| PDIA1 (ng/ml), mean (SD), [95% CI] | 2.55 (0.44), [2.37-2.74] | 2.81 (0.67), [2.56-3.07] |
| PDIA3* (ng/ml), mean (SD), [95% CI] | 0.098 (0.054), [0.074-0.121] | 0.078 (0.071), [0.051-0.105] |
| MANF (ng/ml), mean (SD), [95% CI] | 0.39 (0.16), [0.32-0.46] | 0.47 (0.24), [0.34-0.56] |
| GRP78 ($\mu$g/ml), mean (SD), [95% CI] | 4.65 (1.34), [4.09-5.21] | 4.82 (1.28), [4.34-5.31] |
| Calreticulin* (ng/ml), mean (SD), [95% CI] | 0.163 (0.047), [0.143-0.183] | 0.172 (0.067), [0.147-0.198] |
| Clusterin ($\mu$g/ml), mean (SD), [95% CI] | 44.12 (7.82), [40.82-47.42] | 54.69 (54.00), [34.14-75.23] |
| $\alpha$-synuclein (ng/ml), mean (SD), [95% CI] | 2.57 (1.21), [2.06-3.07] | 2.84 (1.80), [2.155-3.53] |
| Oligo $\alpha$-synuclein (pg/ml), mean (SD), [95% CI] | 11.49 (22.03), [2.18-20.79] | 22.01 (61.97), [-1.56-45.58] |

Mean biomarker concentrations, standard deviations (SD) and 95% confidence intervals (CI) are given in serum of non-Parkinson's disease (PD) and PD diagnostic groups. *23 non-PD samples.

**Panel of biomarkers discriminates PD patients from control group**

To evaluate if the combination of multiple biomarkers allows for diagnosis of PD we performed a multiple logistic regression analysis and identified the differentiating set of biomarkers. The panel of four biomarkers PDIA1, PDIA3, MANF, and clusterin together with two confounders age and gender allowed for discrimination of PD patients from non-PD control group, with an area under the curve (AUC) 0.64.

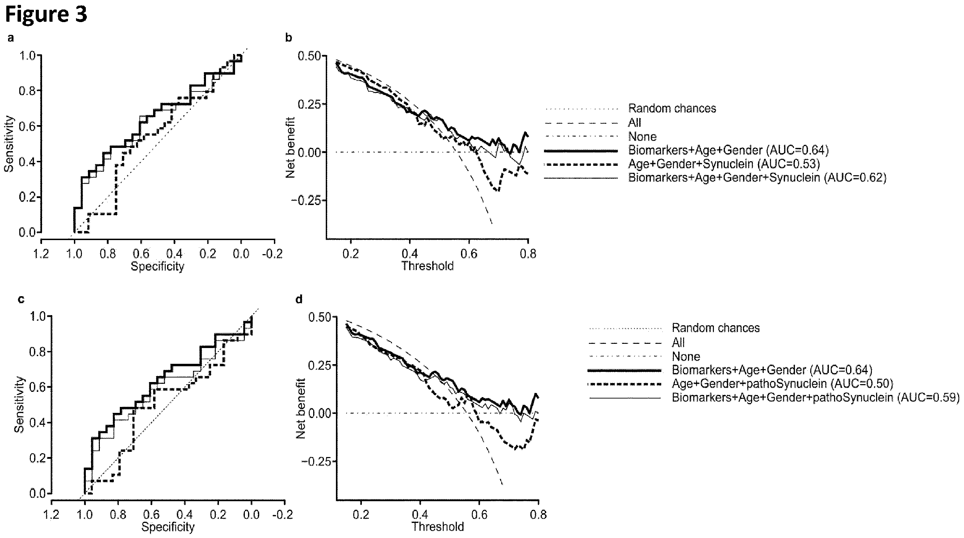

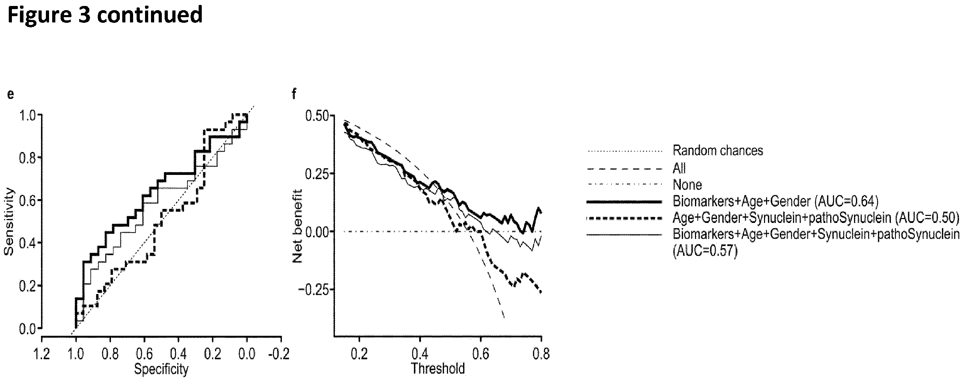

Combining biomarkers reflecting different neuropathological processes in PD may improve a diagnostic accuracy. Conversion of monomeric $\alpha$-synuclein into oligomers and fibrils is a major neuropathological hallmark of PD and has gained an attention as a promising diagnostic biomarker. We measured levels of pathological and total $\alpha$-synuclein

in serum (Table 2) and performed a logistic regression analysis to test if α-synuclein can contribute to our biomarker panel performance. A combined assessment revealed that neither pathological α-synuclein nor total α-synuclein improved a diagnostic discriminatory power and benefit of our biomarker panel (Figure 3).

**Discussion:**

This exploratory study is the first showing performance of ER stress-regulated chaperones in diagnosis of PD. We demonstrate that levels of PDIA1, PDIA3, MANF and clusterin in serum can serve as a biomarker panel to discriminate PD patients from non-PD control group with levels of PDIA1 and MANF contributing most to the diagnostic accuracy.

This study confirms that chaperones normally retained in ER can be detected extracellularly in circulation. PDIA1, PDIA3, MANF, GRP78 and calreticulin are ER resident chaperones retained in ER under normal conditions due to C-terminal Lys-Asp-Glu-Leu (KDEL)-like sequence. When they escape to the Golgi, they are recognized by KDEL receptors and transported back to the ER lumen [25]. It is believed that upon ER stress those chaperones can be released from cells due to saturation of KDEL receptors [26]. However, this theory does not explain why not all KDEL proteins escape the retrieval pathway upon ER stress [27][28]. It appears that the secretion of ER resident chaperones is somehow selective. One explanation might be the differences in retention sequences between chaperones. MANF has a C-terminal RTDL sequence, which fosters weaker ER retention compared to KDEL sequence [29]. Chaperones can also be cosecreted in complexes with protein partners and escape ER due to hindered KDEL motif [30]. It is therefore most likely that changes in levels of ER resident chaperones observed in blood reflect systemic ER stress.

Fluctuations of chaperone levels in extracellular compartments have been observed in several disorders associated with ER stress. PDIA1 has been proposed as a cerebrospinal fluid (CSF) biomarker of ALS [31]. Elevated plasma levels of MANF and PDIA3 have been observed in colorectal cancer [32]. Increased concentration of clusterin in plasma was predictive of Alzheimer's disease [33]. This highlights limitations of using each of those proteins as a satisfactory single biomarker to discriminate between diseases. We believe that combination of multiple biomarkers is a more reliable approach. Our panel of biomarkers allowed us to discriminate between PD and non-PD groups. However, it is essential to evaluate the model in larger cohorts of patients representing a spectrum of ER stress-related disorders to confirm its specificity and selectivity in diagnosis of PD. Longitudinal studies are needed to assess the dynamics of chaperone level changes as a function of PD stage and duration.

Combining biomarkers reflecting multiple neuropathological processes occurring in PD may improve a diagnostic accuracy of the panel. Biomarkers of inflammation, neurodegeneration, lysosomal dysfunction, metabolic impairment, amyloid pathology, tauopathy and synucleinopathy were investigated so far [34]. Here, we demonstrate that biomarkers of ER stress should be further assessed for their contribution to multiple biomarkers of PD pathological processes to identify the powerful diagnostic set of biomarkers. We determined that inclusion of total α-synuclein and pathological α-synuclein to our model did not improve performance of biomarker panel in discriminating PD patients from non-PD controls. We did not observe significant differences of total α-synuclein levels between diagnostic groups. The reported levels of total α-synuclein in blood of PD patients and controls are contradictory, raising an issue concerning sample contamination with α-synuclein from hemolyzed red blood cells [35][36][37].

Oligomeric α-synuclein is a disease protein variant, therefore should be more reliable compared to total α-synuclein. Single small cohort study suggested that elevated levels of α-synuclein oligomers in serum might be a powerful biomarker to diagnose PD [38]. Contradictory to this study we did not observe a significant differences of α-synuclein oligomers between PD patients and control subjects. The reason for that could be in different capture antibody used in ELISA assay, 5G4, which might detect only a subset of oligomeric forms of α-synuclein possibly present in serum at a very low level [39] [40]. The pathological α-synuclein may assemble into structurally distinct oligomers of different molecular weights [41]. 5G4 antibody was generated by immunization of mice with TKEGVVHGVATVAE peptide which recognizes epitope exposed only when α-synuclein is in a β-sheet conformation characteristic for fibrils of α-synuclein. Authors reported that 5G4 antibody can detect α-synuclein fibrils and a dimer [40]. However, α-synuclein oligomers adopt an antiparallel β-sheet structure which might not be recognized by 5G4 antibody [42].

Here we report that a panel of four ER stress-regulated biomarkers discriminates PD from controls. Although the diagnostic accuracy was moderate at this combination, we believe that further validation would lead to a better understanding of biomarkers dynamics in relation to disease stage and severity and will allow to identify the optimal diagnostic biomarker combination with an important benefit for PD patients.

**Example 2: Example analysis**

The levels of markers are analysed and may be presented in a format according to Table 3 below.

Table 3: Example results table.

| | Estimate | Odds Ratio | Std. Error | Z value | Pr(>\|z\|) |
|---|---|---|---|---|---|
| (Intercept) | -5.9197 | 0.0027 | 2.8814 | -2.05 | 0.04* |
| Protein disulphide isomerase A1 | 1.2402 | 3.4563 | 0.6592 | 1.88 | 0.05. |
| Clusterin | 0.01192 | 1.012 | 0.0202 | 0.59 | 0.55 |
| Mesencephalic astrocyte-derived neurotrophic factor | 4.2073 | 67.17 | 1.9822 | 2.12 | 0.03* |
| Protein disulphide isomerase A3 | -4.2548 | 0.0142 | 5.1384 | -0.83 | 0.41 |
| Age | 0.0264 | 1.0268 | 0.0320 | 0.82 | 0.41 |
| Gender(Female) | -1.5851 | 0.2049 | 0.7221 | -2.19 | 0.03* |

The odds of PD for Female is 80% lower than a male patient, and for every one unit increase in protein E (MANF), the odds of PD will increase by 6617%. In addition, for every one unit increase in protein A (PDIA1), the odds of PD will increase by 245%. For every one unit increase in protein C (clusterin), the odds of PD will increase by 1%. For every one unit increase in protein F (PDIA3), the odds of PD will decrease by 98%. For every one year increase in Age, the odds of PD will increase by 2%.

Once the biomarker levels are measured for a specific patient, the following model can be used to estimate the risk of PD.

$$P(PD)$$
$$= \frac{\exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}{1 + \exp\left(-5.9197 + 1.2402\ Protein\ A + 0.01192\ Protein\ C + 4.2073\ Protein\ E - 4.2548\ Protein\ F + 0.0264\ Age - 1.5851\ Gender\right)}$$

In the above formulae, Gender is 1 if the patient is female, and 0 for male.

**References:**

All references are incorporated by reference herein.

[1] W. Poewe et al., "Parkinson disease," Nat. Rev. Dis. Prim., vol. 3, no. 1, pp. 1-21, Mar. 2017.

[2] J. J. M. Hoozemans, E. S. van Haastert, P. Eikelenboom, R. A. I. de Vos, J. M. Rozemuller, and W. Scheper, "Activation of the unfolded protein response in Parkinson's disease," Biochem. Biophys. Res. Commun., vol. 354, no. 3, pp. 707-711, Mar. 2007.

[3] S. Selvaraj et al., "Neurotoxin-induced ER stress in mouse dopaminergic neurons involves downregulation of TRPC1 and inhibition of AKT/mTOR signaling.," J. Clin. Invest., vol. 122, no. 4, pp. 1354-67, Apr. 2012.

[4] S. M. Heman-Ackah et al., "Alpha-synuclein induces the unfolded protein response in Parkinson's disease SNCA triplication iPSC-derived neurons.," Hum. Mol. Genet., vol. 26, no. 22, pp. 4441-4450, 2017.

[5] K. J. Conn et al., "Identification of the protein disulfide isomerase family member PDIp in experimental Parkinson's disease and Lewy body pathology," Brain Res., vol. 1022, no. 1-2, pp. 164-172, Oct. 2004.

[6] E. Colla et al., "Endoplasmic reticulum stress is important for the manifestations of α-synucleinopathy in vivo.," J. Neurosci., vol. 32, no. 10, pp. 3306-20, Mar. 2012.

[7] L. Plate and R. L. Wiseman, "Regulating Secretory Proteostasis through the Unfolded Protein Response: From Function to Therapy," Trends Cell Biol., vol. 27, no. 10, pp. 722-737, Oct. 2017.

[8] C. Hetz and R. J. Kaufman, "Mechanisms, regulation and functions of the unfolded protein response," Nat. Rev. Mol. Cell Biol., pp. 1-18, May 2020.

[9] G. Mercado, P. Valdés, and C. Hetz, "An ERcentric view of Parkinson's disease," Trends in Molecular Medicine, vol. 19, no. 3. Elsevier Current Trends, pp. 165-175, 01-Mar-2013.

[10] P. Nizard et al., "Stress-Induced Retrotranslocation of Clusterin/ApoJ into the Cytosol," Traffic, vol. 8, no. 5, pp. 554-565, May 2007.

[11] R. C. Taylor and A. Dillin, "XBP-1 Is a Cell-Nonautonomous Regulator of Stress Resistance and Longevity," Cell, vol. 153, no. 7, pp. 1435-1447, Jun. 2013.

[12] N. T. Sprenkle, A. Lahiri, J. W. Simpkins, and G. P. Meares, "Endoplasmic reticulum stress is transmissible in vitro between cells of the central nervous system," J. Neurochem., vol. 148, no. 4, pp. 516-530, Feb. 2019.

[13] K. W. Williams et al., "Xbpls in Pomc Neurons Connects ER Stress with Energy Balance and Glucose Homeostasis," CellMetab., vol. 20, no. 3, pp. 471-482, Sep. 2014.

[14] Y.-T. Sui, K. M. Bullock, M. A. Erickson, J. Zhang, and W. A. Banks, "Alpha synuclein is transported into and out of the brain by the blood-brain barrier," Peptides, vol. 62, pp. 197-202, Dec. 2014.

[15] O. M. A. El-Agnaf et al., "Detection of oligomeric forms of $\alpha$-synuclein protein in human plasma as a potential biomarker for Parkinson's disease," FASEB J., vol. 20, no. 3, pp. 419-425, Mar. 2006.

[16] B. D. Jovanovic, "Subset selection in regression. A. J. Miller, Chapman and Hall, London, 1990. No. of pages: x + 229. Price: £25," Stat. Med., vol. 10, no. 7, pp. 1164-1165, Jul. 1991.

[17] D. J. Spiegelhalter, "Probabilistic prediction in patient management and clinical trials," Stat. Med., vol. 5, no. 5, pp. 421-433, Sep. 1986.

[18] R. R. Picard and R. D. Cook, "Cross-validation of regression models," J. Am. Stat. Assoc., vol. 79, no. 387, pp. 575-583, 1984.

[19] J. A. Hanley and B. J. McNeil, "The meaning and use of the area under a receiver operating characteristic (ROC) curve," Radiology, vol. 143, no. 1, pp. 29-36, 1982.

[20] A. C. Cohen, "Simplified Estimators for the Normal Distribution When Samples Are Singly Censored or Truncated," Technometrics, vol. 1, no. 3, p. 217, Aug. 1959.

[21] L. I. Gold et al., "Calreticulin: non-endoplasmic reticulum functions in physiology and disease," FASEB J., vol. 24, no. 3, pp. 665-683, Mar. 2010.

[22] C. Turano, S. Coppari, F. Altieri, and A. Ferraro, "Proteins of the PDI family: Unpredicted non-ER locations and functions," J. Cell. Physiol., vol. 193, no. 2, pp. 154-163, Nov. 2002.

[23] M. H. Voutilainen, U. Arumäe, M. Airavaara, and M. Saarma, "Therapeutic potential of the endoplasmic reticulum located and secreted CDNF/MANF family of neurotrophic factors in Parkinson's disease," FEBS Lett., vol. 589, no. 24PartA, pp. 3739-3748, Dec. 2015.

[24] M. Ni, Y. Zhang, and A. S. Lee, "Beyond the endoplasmic reticulum: Atypical GRP78 in cell viability, signalling and therapeutic targeting," Biochemical Journal, vol. 434, no. 2. NIH Public Access, pp. 181-188, 01-Mar-2011.

[25] P. Bräuer et al., "Structural basis for pH-dependent retrieval of ER proteins from the Golgi by the KDEL receptor," Science (80-. )., vol. 363, no. 6431, pp. 1103-1107, Mar. 2019.

[26] K. A. Trychta, S. Back, M. J. Henderson, and B. K. Harvey, "KDEL Receptors Are Differentially Regulated to Maintain the ER Proteome under Calcium Deficiency," Cell Rep., vol. 25, no. 7, pp. 1829-1840.e6, Nov. 2018.

[27] G. Xiao, T. F. Chung, H. Y. Pyun, R. E. Fine, and R. J. Johnson, "KDEL proteins are found on the surface of NG108-15 cells," Mol. Brain Res., vol. 72, no. 2, pp. 121-128, Oct. 1999.

[28] D. L. Wiest, A. Bhandoola, J. Punt, G. Kreibich, D. Mckean, and A. Singer, "Incomplete endoplasmic reticulum (ER) retention in immature thymocytes as revealed by surface expression of 'ER-resident' molecular chaperones," Proc. Natl. Acad. Sci. U. S. A., vol. 94, no. 5, pp. 1884-1889, Mar. 1997.

[29] M. J. Henderson, C. T. Richie, M. Airavaara, Y. Wang, and B. K. Harvey, "Mesencephalic astrocyte-derived neurotrophic factor (MANF) secretion and cell surface binding are modulated by KDEL receptors," J. Biol. Chem., vol. 288, no. 6, pp. 4209-4225, Feb. 2013.

[30] U. K. Misra, M. Gonzalez-Gronow, G. Gawdi, and S. V. Pizzo, " The Role of MTJ-1 in Cell Surface Translocation of GRP78, a Receptor for $\alpha$ 2 -Macroglobulin-Dependent Signaling ," J. Immunol., vol. 174, no. 4, pp. 2092-2097, Feb. 2005.

[31] J. D. Atkin, M. A. Farg, A. K. Walker, C. McLean, D. Tomas, and M. K. Horne, "Endoplasmic reticulum stress and induction of the unfolded protein response in human sporadic amyotrophic lateral sclerosis," Neurobiol. Dis., vol. 30, no. 3, pp. 400-407, Jun. 2008.

[32] J. J. Ladd et al., "Increased plasma levels of the APC-interacting protein MAPRE1, LRG1, and IGFBP2 preceding a diagnosis of colorectal cancer in women," Cancer Prev. Res., vol. 5, no. 4, pp. 655-664, Apr. 2012.

[33] M. Thambisetty et al., "Association of plasma clusterin concentration with severity, pathology, and progression in Alzheimer disease," Arch. Gen. Psychiatry, vol. 67, no. 7, pp. 739-748, Jul. 2010.

[34] L. Parnetti et al., "CSF and blood biomarkers for Parkinson's disease," The Lancet Neurology, vol. 18, no. 6. Lancet Publishing Group, pp. 573-586, 01-Jun-2019.

[35] H. L. Wang et al., "Combined assessment of serum alpha-synuclein and RAB35 is a better biomarker for Parkinson's disease," J. Clin. Neurol., vol. 15, no. 4, pp. 488-495, Oct. 2019.

[36] C.-W. Chang, S.-Y. Yang, C.-C. Yang, C.-W. Chang, and Y.-R. Wu, "Plasma and Serum Alpha-Synuclein as a Biomarker of Diagnosis in Patients With Parkinson's Disease," Front. Neurol., vol. 10, p. 1388, Jan. 2020.

[37] R. Barbour et al., "Red blood cells are the major source of alpha-synuclein in blood," Neurodegener. Dis., vol. 5, no. 2, pp. 55-59, Jan. 2008.

[38] S. M. Williams, P. Schulz, and M. R. Sierks, "Oligomeric $\alpha$-synuclein and $\beta$-amyloid variants as potential biomarkers for Parkinson's and Alzheimer's diseases," Eur. J. Neurosci., vol. 43, no. 1, pp. 3-16, Jan. 2016.

[39] S. Emadi, S. Kasturirangan, M. S. Wang, P. Schulz, and M. R. Sierks, "Detecting morphologically distinct oligomeric forms of $\alpha$-synuclein," J. Biol. Chem., vol. 284, no. 17, pp. 11048-11058, Apr. 2009.

G. G. Kovacs et al., "An antibody with high reactivity for disease-associated α-synuclein reveals extensive brain pathology," Acta Neuropathol., vol. 124, no. 1, pp. 37-50, Jul. 2012.

P. Alam, L. Bousset, R. Melki, and D. E. Otzen, "α-synuclein oligomers and fibrils: a spectrum of species, a spectrum of toxicities," J. Neurochem., vol. 150, no. 5, pp. 522-534, Sep. 2019.

M. Soledad Celej, R. Sarroukh, E. Goormaghtigh, G. Fidelio, J.-M. Ruysschaert, and V. Raussens, "Alpha-Synuclein Amyloid Oligomers Exhibit Beta-Sheet Antiparallel Structure as Revealed by FTIR Spectroscopy," Biophys. J., vol. 102, no. 3, pp. 440a-441a, Jan. 2012.

R. B. Postuma et al., "MDS clinical diagnostic criteria for Parkinson's disease" Movement Disorders, vol. 30, no. 12, pp. 1591-1601, Oct. 2015.

M. M. Hoehn & M. D. Yahr, "Parkinsonism: onset, progression and mortality" Neurology, vol. 17, no. 5, pp. 427-442, May 1967.

**Claims**

1. A method of determining whether an individual has Parkinson's disease, the method comprising:

   determining the level of markers in a bodily fluid sample from the individual, wherein the markers comprise Protein disulphide isomerase A1 (PDIA1), Protein disulphide isomerase A3 (PDIA3), Mesencephalic astrocyte-derived neurotrophic factor (MANF) and clusterin;

   wherein an increase in the level of PDIA1, MANF, and clusterin, and decrease in the level of PDIA3 relative to a reference level in combination with the individual's age and gender, is indicative of the individual having Parkinson's disease.

2. The method according to claim 1, wherein a probability that an individual has Parkinson's disease is determined by the calculation:

$$P(PD) = \frac{\exp\left(-5.9197 + 1.2402\, Protein\ A + 0.01192\, Protein\ C + 4.2073\, Protein\ E - 4.2548\, Protein\ F + 0.0264\, Age - 1.5851\, Gender\right)}{1 + \exp\left(-5.9197 + 1.2402\, Protein\ A + 0.01192\, Protein\ C + 4.2073\, Protein\ E - 4.2548\, Protein\ F + 0.0264\, Age - 1.5851\, Gender\right)}$$

   wherein Protein A is PDIA1, Protein C is clusterin, Protein E is MANF and Protein F is PDIA3 and these proteins are the measured biomarker levels, age is the individual's age and gender is assigned 1 for female or 0 for male.

3. The method of claim 1 or 2, wherein the individual is exhibiting clinical features of Parkinson's disease; and/or wherein the individual is exhibiting symptoms at Hoehn and Yahr stage 2 or 3 of Parkinson's disease.

4. The method according to any preceding claim, wherein the method further comprises the assessment of clinical features of Parkinson's disease.

5. The method according to any of claims 3 or 4, wherein the clinical features comprise bradykinesia in combination with one or more, or all, of the clinical features selected from the group comprising: rest tremor, rigidity, postural instability, hypomimia, dysarthria, dysphagia, sialorrhoea, decreased arm swing, shuffling gait, festination, difficulty arising from chair, difficulty turning in bed, micrographia, difficulty cutting food, reduced hygiene, slow activities of daily living, glabellar reflex, blepharospasm, dystonia, striatal deformity, scoliosis, camptocormia, cognitive impairment, bradyphrenia, tip-of-the-tongue phenomenon, depression, apathy, anhedonia, fatigue, other behavioural and psychiatric problems, sensory symptoms (such as anosmia, ageusia, pain (shoulder, back) or paresthesias), dysautonomia (such as orthostatic hypotension, constipation, urinary and sexual dysfunction, abnormal sweating, seborrhoea), weight loss, sleep disorders (such as REM behaviour disorder, vivid dreams, daytime drowsiness, sleep fragmentation, restless legs syndrome), and/or response to high-dose levodopa.

6. The method according to any preceding claim, wherein the method further comprises selecting the individual for therapeutic intervention and/or follow-up check, if the individual is diagnosed with Parkinson's disease.

7. The method according to any preceding claim, wherein the method further comprises administering a therapeutic or preventative medication to the individual, if the individual is diagnosed with Parkinson's disease; and optionally the method further comprises providing supportive therapies including one or more of physiotherapy, occupational therapy, speech and language therapy and diet advice, if the individual is diagnosed with Parkinson's disease; and further optionally the method further comprises referring the individual for, or conducting, surgery arranged to alleviate some of the symptoms of Parkinson's disease (such as deep brain stimulation), if the individual is diagnosed with Parkinson's disease.

8. The method according to claim 7, wherein the therapeutic or preventative medication comprises one or more of dopamine receptor activators, dopamine precursors, inhibitors of dopamine degradation, or indirect dopamine receptor activators.

9. The method according to any preceding claim, wherein determining the level of a marker comprises binding a marker with one or more probes; and optionally further comprises detecting the act of binding of the probe(s) to the marker or detecting the level of bound probe-marker complexes; optionally wherein the probe comprises an antibody, an antibody variant or memetic, or a binding-fragment thereof, or the probe comprises an aptamer.

10. The method according to any of claim 9, wherein the probe(s) are immobilised on a substrate.

11. The method according to any preceding claim, wherein the bodily fluid sample is selected from a blood sample, a serum or a plasma sample, a cerebrospinal fluid sample, a saliva sample, a mucus sample, and a urine sample.

12. A composition comprising a plurality of probes capable of binding to protein markers in a bodily fluid sample, wherein the protein markers comprise PDIA1, PDIA3, MANF, and clusterin, optionally wherein the probes are provided as a panel of probes anchored to a surface.

13. A method for treating an individual with Parkinson's disease, the method comprising the steps of:

determining whether the individual has Parkinson's disease by:
obtaining or having obtained a sample from the individual; and performing or having performed the method according to any of claims 1-15 to determine if the individual has Parkinson's disease; and
if the individual is found to have Parkinson's disease, then administering medication to the individual and/or surgically treating the individual.

14. A method for treating an individual diagnosed with Parkinson's disease, the method comprising the steps of:

receiving results of a test performed according to the method according to any of the claims 1-15 to determine if the individual has Parkinson's disease; and
if the individual is found to have Parkinson's disease, then administering medication as described herein to the individual and/or surgically treating the individual.

15. Use of PDIA1, PDIA3, MANF and clusterin, proteins as markers in a bodily fluid sample from an individual for the diagnosis of Parkinson's disease, optionally wherein the markers are used in correlation with the age and gender of the individual.

Figure 1

EP 4 242 662 A1

**Figure 2**

**Figure 3**

**Figure 3 continued**

Europäisches
Patentamt

European
Patent Office

Office européen
des brevets

# EUROPEAN SEARCH REPORT

**Application Number**

EP 22 16 1689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG Z ET AL: "An array of 60,000 antibodies for proteome-scale antibody generation and target discovery", SCI. ADV., vol. 6, no. eaax2271, 1 January 2020 (2020-01-01), XP55949649, Retrieved from the Internet: URL:https://www.science.org/doi/pdf/10.1126/sciadv.aax2271> | 12 | INV. G01N33/68 |
| A | * the whole document * <br> * abstract * | 1-11, 13-15 | |
| X | ARMSTRONG MELISSA J ET AL: "Diagnosis and Treatment of Parkinson Disease A Review", JAMA THE JOURNAL OF THE AMERICAN MEDICAL ASSOCIATION, AMERICAN MEDICAL ASSOCIATION, US, vol. 323, no. 6, 11 February 2020 (2020-02-11), pages 548-560, XP009524499, ISSN: 0098-7484, DOI: 10.1001/JAMA.2019.22360 | 13,14 | |
| A | * the whole document * <br> * abstract * | 1-12,15 | **TECHNICAL FIELDS SEARCHED (IPC)** <br> G01N |
| Y | CONN K J ET AL: "Identification of the protein disulfide isomerase family member PDIp in experimental Parkinson's disease and Lewy body pathology", BRAIN RESEARCH, ELSEVIER, AMSTERDAM, NL, vol. 1022, no. 1-2, 1 October 2004 (2004-10-01), pages 164-172, XP004558752, ISSN: 0006-8993, DOI: 10.1016/J.BRAINRES.2004.07.026 | 12 | |
| A | * the whole document * <br> * abstract * | 1-11, 13-15 | |

-/--

The present search report has been drawn up for all claims

| Place of search | Date of completion of the search | Examiner |
|---|---|---|
| The Hague | 8 August 2022 | Jenkins, Gareth |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

# EUROPEAN SEARCH REPORT

Application Number

EP 22 16 1689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GALLI EMILIA ET AL: "Increased Serum Levels of Mesencephalic Astrocyte-Derived Neurotrophic Factor in Subjects With Parkinson's Disease", FRONTIERS IN NEUROSCIENCE, vol. 13, 4 September 2019 (2019-09-04), XP55949664, DOI: 10.3389/fnins.2019.00929 | 12 | |
| A | * the whole document * <br> * abstract * | 1-11, 13-15 | |
| Y | POLIMENO LORENZO ET AL: "Plasma levels of Clusterin are representative of the early phase of the neurodegenerative process in Parkinson's disease", JOURNAL OF CLINICAL AND MOLECULAR MEDICINE, vol. 1, no. 1, 1 January 2018 (2018-01-01), XP55949666, DOI: 10.15761/JCMM.1000102 Retrieved from the Internet: URL:https://www.oatext.com/pdf/JCMM-1-102.pdf> | 12 | |
| | | | TECHNICAL FIELDS SEARCHED (IPC) |
| A | * the whole document * <br> * abstract * | 1-11, 13-15 | |
| Y | US 2017/335395 A1 (CHEN-PLOTKIN ALICE [US] ET AL) 23 November 2017 (2017-11-23) | 12 | |
| A | * the whole document * <br> * table 3 * | 1-11, 13-15 | |

The present search report has been drawn up for all claims

| Place of search | Date of completion of the search | Examiner |
|---|---|---|
| The Hague | 8 August 2022 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 1689

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2022

| Patent document cited in search report | | Publication date | Patent family member(s) | | Publication date |
|---|---|---|---|---|---|
| US 2017335395 | A1 | 23-11-2017 | US | 2017335395 A1 | 23-11-2017 |
| | | | WO | 2016069461 A1 | 06-05-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **W. POEWE et al.** Parkinson disease. *Nat. Rev. Dis. Prim.,* March 2017, vol. 3 (1), 1-21
- **J. J. M. HOOZEMANS ; E. S. VAN HAASTERT ; P. EIKELENBOOM ; R. A. I. DE VOS ; J. M. ROZEMULLER ; W. SCHEPER.** Activation of the unfolded protein response in Parkinson's disease. *Biochem. Biophys. Res. Commun.,* March 2007, vol. 354 (3), 707-711
- **S. SELVARAJ et al.** Neurotoxin-induced ER stress in mouse dopaminergic neurons involves downregulation of TRPC1 and inhibition of AKT/mTOR signaling. *J. Clin. Invest.,* April 2012, vol. 122 (4), 1354-67
- **S. M. HEMAN-ACKAH et al.** Alpha-synuclein induces the unfolded protein response in Parkinson's disease SNCA triplication iPSC-derived neurons. *Hum. Mol. Genet.,* 2017, vol. 26 (22), 4441-4450
- **K. J. CONN et al.** Identification of the protein disulfide isomerase family member PDIp in experimental Parkinson's disease and Lewy body pathology. *Brain Res.,* October 2004, vol. 1022 (1-2), 164-172
- **E. COLLA et al.** Endoplasmic reticulum stress is important for the manifestations of $\alpha$-synucleinopathy in vivo. *J. Neurosci.,* March 2012, vol. 32 (10), 3306-20
- **L. PLATE ; R. L. WISEMAN.** Regulating Secretory Proteostasis through the Unfolded Protein Response: From Function to Therapy. *Trends Cell Biol.,* October 2017, vol. 27 (10), 722-737
- **C. HETZ ; R. J. KAUFMAN.** Mechanisms, regulation and functions of the unfolded protein response. *Nat. Rev. Mol. Cell Biol.,* May 2020, 1-18
- An ERcentric view of Parkinson's disease. **G. MERCADO ; P. VALDÉS ; C. HETZ.** Trends in Molecular Medicine. Elsevier Current Trends, 01 March 2013, vol. 19, 165-175
- **P. NIZARD et al.** Stress-Induced Retrotranslocation of Clusterin/ApoJ into the Cytosol. *Traffic,* May 2007, vol. 8 (5), 554-565
- **R. C. TAYLOR ; A. DILLIN.** XBP-1 Is a Cell-Nonautonomous Regulator of Stress Resistance and Longevity. *Cell,* June 2013, vol. 153 (7), 1435-1447
- **N. T. SPRENKLE ; A. LAHIRI ; J. W. SIMPKINS ; G. P. MEARES.** Endoplasmic reticulum stress is transmissible in vitro between cells of the central nervous system. *J. Neurochem.,* February 2019, vol. 148 (4), 516-530

- **K. W. WILLIAMS et al.** Xbpls in Pomc Neurons Connects ER Stress with Energy Balance and Glucose Homeostasis. *CellMetab.,* September 2014, vol. 20 (3), 471-482
- **Y.-T. SUI ; K. M. BULLOCK ; M. A. ERICKSON ; J. ZHANG ; W. A. BANKS.** Alpha synuclein is transported into and out of the brain by the blood-brain barrier. *Peptides,* December 2014, vol. 62, 197-202
- **O. M. A. EL-AGNAF et al.** Detection of oligomeric forms of $\alpha$-synuclein protein in human plasma as a potential biomarker for Parkinson's disease. *FASEB J.,* March 2006, vol. 20 (3), 419-425
- Subset selection in regression. **B. D. JOVANOVIC.** Stat. Med. A. J. Miller, Chapman and Hall, 1990, vol. 10, 1164-1165
- **D. J. SPIEGELHALTER.** Probabilistic prediction in patient management and clinical trials. *Stat. Med.,* September 1986, vol. 5 (5), 421-433
- **R. R. PICARD ; R. D. COOK.** Cross-validation of regression models. *J. Am. Stat. Assoc.,* 1984, vol. 79 (387), 575-583
- **J. A. HANLEY ; B. J. MCNEIL.** The meaning and use of the area under a receiver operating characteristic (ROC) curve. *Radiology,* 1982, vol. 143 (1), 29-36
- **A. C. COHEN.** Simplified Estimators for the Normal Distribution When Samples Are Singly Censored or Truncated. *Technometrics,* August 1959, vol. 1 (3), 217
- **L. I. GOLD et al.** Calreticulin: non-endoplasmic reticulum functions in physiology and disease. *FASEB J.,* March 2010, vol. 24 (3), 665-683
- **C. TURANO ; S. COPPARI ; F. ALTIERI ; A. FERRARO.** Proteins of the PDI family: Unpredicted non-ER locations and functions. *J. Cell. Physiol.,* November 2002, vol. 193 (2), 154-163
- **M. H. VOUTILAINEN ; U. ARUMÄE ; M. AIRAVAARA ; M. SAARMA.** Therapeutic potential of the endoplasmic reticulum located and secreted CDNF/MANF family of neurotrophic factors in Parkinson's disease. *FEBS Lett.,* December 2015, vol. 589 (24), 3739-3748
- **M. NI ; Y. ZHANG ; A. S. LEE.** Beyond the endoplasmic reticulum: Atypical GRP78 in cell viability, signalling and therapeutic targeting. *Biochemical Journal,* 01 March 2011, vol. 434 (2), 181-188

- **P. BRÄUER et al.** Structural basis for pH-dependent retrieval of ER proteins from the Golgi by the KDEL receptor. *Science,* March 2019, vol. 363 (6431), 1103-1107
- **K. A. TRYCHTA ; S. BACK ; M. J. HENDERSON ; B. K. HARVEY.** KDEL Receptors Are Differentially Regulated to Maintain the ER Proteome under Calcium Deficiency. *Cell Rep.,* November 2018, vol. 25 (7), 1829-1840.e6
- **G. XIAO ; T. F. CHUNG ; H. Y. PYUN ; R. E. FINE ; R. J. JOHNSON.** KDEL proteins are found on the surface of NG108-15 cells. *Mol. Brain Res.,* October 1999, vol. 72 (2), 121-128
- **D. L. WIEST ; A. BHANDOOLA ; J. PUNT ; G. KREIBICH ; D. MCKEAN ; A. SINGER.** Incomplete endoplasmic reticulum (ER) retention in immature thymocytes as revealed by surface expression of 'ER-resident' molecular chaperones. *Proc. Natl. Acad. Sci. U. S. A.,* March 1997, vol. 94 (5), 1884-1889
- **M. J. HENDERSON ; C. T. RICHIE ; M. AIRAVAARA ; Y. WANG ; B. K. HARVEY.** Mesencephalic astrocyte-derived neurotrophic factor (MANF) secretion and cell surface binding are modulated by KDEL receptors. *J. Biol. Chem.,* February 2013, vol. 288 (6), 4209-4225
- **U. K. MISRA ; M. GONZALEZ-GRONOW ; G. GAWDI ; S. V. PIZZO.** The Role of MTJ-1 in Cell Surface Translocation of GRP78, a Receptor for $\alpha$ 2 -Macroglobulin-Dependent Signaling. *J. Immunol.,* February 2005, vol. 174 (4), 2092-2097
- **J. D. ATKIN ; M. A. FARG ; A. K. WALKER ; C. MCLEAN ; D. TOMAS ; M. K. HORNE.** Endoplasmic reticulum stress and induction of the unfolded protein response in human sporadic amyotrophic lateral sclerosis. *Neurobiol. Dis.,* June 2008, vol. 30 (3), 400-407
- **J. J. LADD et al.** Increased plasma levels of the APC-interacting protein MAPRE1, LRG1, and IGFBP2 preceding a diagnosis of colorectal cancer in women. *Cancer Prev. Res.,* April 2012, vol. 5 (4), 655-664
- **M. THAMBISETTY et al.** Association of plasma clusterin concentration with severity, pathology, and progression in Alzheimer disease. *Arch. Gen. Psychiatry,* July 2010, vol. 67 (7), 739-748
- CSF and blood biomarkers for Parkinson's disease. **L. PARNETTI et al.** The Lancet Neurology. Lancet Publishing Group, 01 June 2019, vol. 18, 573-586
- **H. L. WANG et al.** Combined assessment of serum alpha-synuclein and RAB35 is a better biomarker for Parkinson's disease. *J. Clin. Neurol.,* October 2019, vol. 15 (4), 488-495
- **C.-W. CHANG ; S.-Y. YANG ; C.-C. YANG ; C.-W. CHANG ; Y.-R. WU.** Plasma and Serum Alpha-Synuclein as a Biomarker of Diagnosis in Patients With Parkinson's Disease. *Front. Neurol.,* January 2020, vol. 10, 1388
- **R. BARBOUR et al.** Red blood cells are the major source of alpha-synuclein in blood. *Neurodegener. Dis.,* January 2008, vol. 5 (2), 55-59
- **S. M. WILLIAMS ; P. SCHULZ ; M. R. SIERKS.** Oligomeric $\alpha$-synuclein and $\beta$-amyloid variants as potential biomarkers for Parkinson's and Alzheimer's diseases. *Eur. J. Neurosci.,* January 2016, vol. 43 (1), 3-16
- **S. EMADI ; S. KASTURIRANGAN ; M. S. WANG ; P. SCHULZ ; M. R. SIERKS.** Detecting morphologically distinct oligomeric forms of $\alpha$-synuclein. *J. Biol. Chem.,* April 2009, vol. 284 (17), 11048-11058
- **G. G. KOVACS et al.** An antibody with high reactivity for disease-associated $\alpha$-synuclein reveals extensive brain pathology. *Acta Neuropathol.,* July 2012, vol. 124 (1), 37-50
- **P. ALAM ; L. BOUSSET ; R. MELKI ; D. E. OTZEN.** $\alpha$-synuclein oligomers and fibrils: a spectrum of species, a spectrum of toxicities. *J. Neurochem.,* September 2019, vol. 150 (5), 522-534
- **M. SOLEDAD CELEJ ; R. SARROUKH ; E. GOORMAGHTIGH ; G. FIDELIO ; J.-M. RUYSSCHAERT ; V. RAUSSENS.** Alpha-Synuclein Amyloid Oligomers Exhibit Beta-Sheet Antiparallel Structure as Revealed by FTIR Spectroscopy. *Biophys. J.,* January 2012, vol. 102 (3), 440a-441a
- **R. B. POSTUMA et al.** MDS clinical diagnostic criteria for Parkinson's disease. *Movement Disorders,* October 2015, vol. 30 (12), 1591-1601
- **M. M. HOEHN ; M. D. YAHR.** Parkinsonism: onset, progression and mortality. *Neurology,* May 1967, vol. 17 (5), 427-442